(19) Europäisches Patentamt

European Patent Office

Office européen des brevets

(11) **EP 2 568 291 A1**

(12) **EUROPEAN PATENT APPLICATION**

(43) Date of publication:
**13.03.2013 Bulletin 2013/11**

(21) Application number: **11007260.0**

(22) Date of filing: **07.09.2011**

(51) Int Cl.:
***G01N 33/68*** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **Roche Diagnostics GmbH
68298 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **Hüdig, Hendrik, Dr.
82377 Penzberg (DE)**
• **Kientsch-Engel, Rosemarie, Dr.
82340 Feldafing (DE)**
• **Rutz, Sandra, Dr.
81377 München (DE)**

(74) Representative: **Freiherr von Campenhausen,
Harald et al
Roche Diagnostics GmbH
Patentabteilung
Sandhofer Straße 116
DE-68305 Mannheim (DE)**

(54) **L-FABP based diagnosis of kidney injury after an acute event or after a surgical intervention**

(57) The present invention provides means and methods for diagnosing acute kidney injury associated after an acute event or after a surgical intervention based on the biomarker L-FABP. In addition, a method of predicting the risk of an individual to suffer from a kidney injury after an acute event or after a surgical intervention in the future is provided as well as corresponding uses and kits.

**Figure 5:**

L-FABP in plasma after cardiac surgery in patients suffering from AKI or not suffering from AKI (No AKI)

## Description

## FIELD OF THE INVENTION

The present invention provides means and methods for early diagnosing kidney injury after an acute event or after a surgical intervention in a blood sample from an individual based on the detection of L-FABP and optional further markers.

Kidney injury represents a complex disorder that occurs in a wide variety of clinical settings, often with serious and fatal complications. Kidney diseases are broadly categorized as chronic kidney disease (CKD) and acute forms of kidney injury. CKD is a chronic disease often resulting from diseases like diabetes, nephropathy, hypertension and cardio-vascular diseases.

Acute forms of kidney injury like acute kidney injury (AKI), in turn, may occur in individuals after an acute event like an accident, a burn or a trauma. Kidney injury is also a major complication after a surgical intervention.

The ability of the kidneys to filter waste is quickly reduced in patients suffering from an acute form of kidney injury, a condition which can then progress to kidney failure. The incidence AKI after a coronary bypass surgery ranges from 10 to 20 % (Mehta et al, Circulation 2006, 114: 2208 - 2216). One to 5 percent of these individuals require postoperative dialysis. The pathogenesis of postoperative AKI is multifactorial and its association with increased morbidity and long term mortality after cardiac surgery is well established. (Brown et al, Annals of Thoracic Surgery 2008, 86: 4-11: Kourliouros et al, European Journal of Cardiothoracic Surgery 2009, in press),

AKI may be prevented in risk patients. Prevention includes careful fluid balance during and after surgery, avoidance of low cardiopulmonary bypass /CPB) perfusion temperatures (Kourliouros et al), avoidance or discontinuation of potentially nephrotoxic drugs prior to surgery or application of drugs such as erythropoietin after surgery (Song et al American Journal of Nephrology 2009, 30: 253 - 260).

Acute kidney injury was so far only diagnosed by assessing the serum creatinine level. An absolute increase in the serum creatinine concentration of larger than 0.3 mg/dL (26,4 $\mu$mol/L) from baseline, or a more than 50 percent increase in the serum creatinine, in a period of 48 hours is indicative for the diagnosis of acute kidney injury. The determination of serum creatinine, however, has the disadvantage that the diagnosis of acute kidney injury can be only diagnosed at late stage of acute kidney injury (approximately 2 days after the acute event).

The marker L-FABP (liver fatty acid binding protein), frequently also referred to as FABP1, is an intracellular carrier protein of free fatty acids that is expressed among others in hepatocytes and in the proximal tubules of the human kidney,

Kamijo et al. (Urinary liver-type fatty acid binding protein as a useful biomarker in chronic kidney disease. Mol Cell Biochem. 2006 Mar; 284(1-2): 175-82) reported that urinary excretion of L-FABP may reflect various kinds of stresses that cause tubulointerstitial damage and may be a useful clinical marker of the progression of chronic renal disease.

Portilla et al. (Kidney International (2008) 73, 465-472) observed in a pediatric population after cardiac surgery an increase in L-FABP in patients developing AKI, but not in those without AKI. A significant increase in serum L-FABP levels was only observed as early as 12 hours after cardiac surgery, In contrast, urinary L-FABP levels measured in the same subset of patients were highly increased within the first 4 hours after surgery when compared to control levels.

Furthermore, Portilla et al. (Portilla et al., The American Journal of Pathology, Vol, 174, p. 1154-1159 (2009) induced different degrees of AKI severity by several renal insults in human L-FABP transgenic mice. Portilla et al. showed that urinary L-FABP levels increased even at 2 hours after insult in cis-platinum induced AKI. However, these data lack clinical validation in humans.

The dissociation between plasma and urine L-FABP levels in AKI were discussed in Devarajan et al. (Informa Healthcare (2008) 2, 387-398), suggesting that increased urinary L-FABP levels at 4 hours after cardiac surgery in AKI patients represent an enhanced shedding of L-FABP from the proximal tubule rather than reflecting increased filtration of high serum levels.

AKI may lead to a number of complications, including metabolic acidosis, high potassium levels, uremia, changes in body fluid balance, and effects to other organ systems. Management of AKI includes supportive care, such as renal replacement therapy, as well as treatment of the underlying disorder. Furthermore, AKI may lead to various complications such metabolic acidosis, high potassium levels, uremia, changes in body fluid balance. The changes in fluid balance may result in heart failure (or may worsen heart failure). Moreover, it may affect other organ systems. In severe cases of a kidney injury, renal replacement therapy (including e.g. hemodialysis or renal transplantation) may be required, In addition, AKI is associated with increased mortality, greater cost, and prolonged Intensive Care Unit (ICU) and hospital stay. The traditional biomarkers of AKI, creatinine and urea, do not detect AKI early. The clinical use of the new early biomarkers is rapidly expanding e.g. during anesthesia and in the ICU. The new biomarkers can be used to evaluate the effect of new techniques and therapeutics on kidney function, as safety markers to monitor toxicity and as measures of treatment effect.

As a result, there is a high need to early diagnose kidney injury in individuals undergoing a surgical intervention or an acute event as early as possible, since the early diagnosis allows the initiation of timely therapeutic measures.

Consequently, one technical problem underlying the present invention could be seen as the provision of means and methods for the early identification of individuals suffering from kidney injury or being at risk to suffer from kidney injury in the future, after an acute event or after a surgical intervention.

## SUMMARY OF THE INVENTION

At least one of these objectives is accomplished by the provision of the subject matter defined in the claims and herein below.

In a first aspect the invention relates to a method for diagnosing a kidney injury in an individual after an acute event or after a surgical intervention comprising the steps of:

a) determining the level of liver-type fatty acid binding protein (L-FABP) or a variant thereof in a first blood sample isolated from an individual, wherein the sample is isolated within about 10 h after the acute event or after the surgical intervention; and
b) comparing the level determined in step a) with a reference level;

wherein a level of L-FABP or a variant thereof which is at least equal to or greater than the reference level is indicative of kidney injury.

In a second aspect the invention relates to a method of predicting the risk of an individual to suffer from a kidney injury after an acute event or after a surgical intervention in the future, comprising the steps of:

a) determining the level of L-FABP or a variant thereof in a first blood sample isolated from an individual, wherein the sample is isolated within 10 h after the event or after the surgical intervention; and
b) comparing the level determined in step a) with a reference level,

wherein a level of L-FABP equal to or greater than the reference level is indicative of a risk of an individual to suffer from a kidney injury.

In a third aspect the invention relates to a method for selecting a renal therapy for an individual suffering from or having a risk to suffer from kidney injury after an acute event or after a surgical intervention comprising the steps of:

a) determining the level of L-FABP or a variant thereof in a first blood sample of an individual isolated within 10 h after the event or after the surgical intervention; and
b) comparing the level of L-FABP determined in step a) with a reference level;

wherein the renal therapy is selected based on the comparison of step b).

In further aspects the invention relates to corresponding kits, and devices and uses thereof

## DETAILED DESCRIPTION OF THE INVENTION

In a first aspect, the present invention relates to a method for diagnosing a kidney injury in an individual after an acute event or after a surgical intervention comprising the steps of:

a) determining the level of liver-type fatty acid binding protein (L-FABF) or a variant thereof in a blood sample isolated from an individual, wherein the sample is isolated within about 10 h after the acute event or after the surgical intervention; and
b) comparing the level determined in step a) with a reference level;

wherein a level of L-FABP or a variant thereof which is at least equal to or greater than the reference level is indicative of kidney injury.

The inventors of the present invention surprisingly found that a blood sample-based detection of L-FABP allows for a hitherto unknown early and robust diagnosis (and prediction) of a kidney injury in an individual after an acute event or after a surgical intervention (see e.g. examples). Such early diagnosis provides the clinician with very valuable information and provides a basis for treatment stratification and for therapeutically intervening at an earlier point in time when compared to hitherto known clinical practice which is essentially based on more unreliable or later reacting marker levels. As a consequence, it is now possible to earlier initiate or adapt therapeutic measures intended to prevent development of kidney injury or ameliorate its deterioration. In this respect, it has to be taken into consideration that after the occurrence of kidney injury, the therapeutical option (options for treatment) are clearly more limited than in cases where the risk of suffering from kidney injury is recognized or its occurrence can be treated prophylactically. Such decisions include reconsideration of the indication for surgery in terms of improving risk benefit assessment, discontinuation of drugs known to precipitate kidney injury such as AKI including ACE inhibitors, angiotensin receptor blockers and NSIADs and potentially antibiotics and other drugs known to precipitate AKI. Furthermore during surgery appropriate and intense balancing of fluid as well as blood pressure is required. Thus the method of the present invention targets prophylaxis of kidney injury thus provides improved clinical decision making.

The methods of the present invention, preferably, are ex vivo or in vitro methods. Moreover, the methods may comprise steps in addition to those explicitly mentioned above. For example, further steps may relate to sample pre-treatments or evaluation of the results obtained by the method. The method may be carried out manually or assisted by automation. Preferably, step (a) and (b) may in total or in part be assisted by automation, e.g., by a suitable robotic and sensory equipment for the determination in step (a), or (b), or a computer-implemented comparison and/or diagnosis based on the comparison in step (b).

Preferably, the kidney injury is diagnosed by carrying out the further step of c) diagnosing the kidney injury based on the result of the comparison of step b). The term "kidney injury" refers to a loss of kidney function, a sub-cellular, cellular damage or a tissue damage of the kidney, need for dialysis and/or kidney malfunction associated death. Preferably, the kidney injury of the present invention is acute kidney injury (AKI). AKI is well known in the art. AKI is preferably defined as an increase of serum creatinine of at least 0.3 mg/dl within 48 hours or within 72h after surgery or by an increase of at least 50 % from baseline (preferably within 48 hours after surgery). AKI may also be diagnosed on the basis of characteristic laboratory findings, such as elevated blood urea nitrogen and creatinine, or based on the inability of the kidneys to produce sufficient amounts of urine. AKI can be a post-renal, intrinsic or, pre-renal injury of the kidney. Further preferred definitions of AKI that are encompassed by the term AKI as used herein have been disclosed by the Acute Kidney Injury Network (AKIN) (www.AKZNet.org) which disclosed a definition of AKI based on the RIFLE classification. Further methods and criteria to diagnose AKI have been described in Mehta et al. (2007) Critical Care (London, England) 11 (2): R31.doi:10.1186/cc5713. PMC 2206446. PMID 17331245).
On the contrary, a chronic kidney injury can be diagnosed by urine secretion of albumin.

The term "kidney function" as used herein is well known to the person skilled in the art. It may be used interchangeably with "renal function" and relates to the capacity of the kidney for urine production, control and elimination of body water and body fluids, and homeostasis and filtration of electrolytes and wastes, and erythropoietin synthesis.

The term "an acute event" as used herein in the context of the present invention preferably refers to any accident, burn, trauma or cancer. The term "accident" includes any and unforeseen and unplanned event or circumstance resulting in damage inflicted on the body by an external force including resulting from fall or physical violence. Preferably the damage interrupts the integrity of the body skin like a wound, preferably a wound that may be associated with by tearing, cutting, piercing, or breaking of the tissue. Preferably the wound has a diameter of at least 3, at least 5, at least 10, at least 15, at least 20 centimeters. Examples of accidents encompassed herein include traffic accidents by vehicles, sport accidents, and work accident. The term "trauma" as used herein encompasses a damage which interrupts the integrity of the body skin like a wound, preferably a wound that may be associated with tearing, cutting, piercing, or breaking of the tissue. Preferably the wound has a diameter of at least 3, at least 5, at least 10, at least 15, at least 20 centimeters.

The term "surgical intervention" as used in the context of the present invention, preferably, refers to any kind of invasive intervention on the body including endoscopic surgical interventions and interventions that involve anesthesia and/or respiratory assistance. Preferably, the anesthesia is localized anesthesia, more preferably, the anesthesia is global anesthesia. The term "surgical intervention", also includes interventions on an inner organ (in particular on the liver, kidney, bowel, stomach, lung). The term "surgical intervention" also includes e.g. interventions on the extremities (legs, arms), and the head. Preferably, a "surgical intervention" according to the present invention is selected from a cardiac surgery or a cardio-pulmonary surgery.

The term "cardiac surgery" includes interventions on the heart, in particular, on the valve or any part of the myocard. Cardiac surgery is, preferably, coronary artery bypass graft (CABG) surgery (frequently also referred to as "aortocoronary bypass" or "coronary artery bypass surgery"). The term CABG is known in the art. CABG may be carried out in an individual suffering from stenosis of the coronary arteries which cannot be treated successfully with other methods such as percutaneous coronary intervention (PCI). The term "cardiac surgery" further includes a valve replacement, a procedure in which an individual's failing valve is replaced with an alternate healthy valve. The valve can be affected by a range of diseases; e.g. the valve can either become leaky (aortic insufficiency/ regurgitation) or partially blocked (aortic stenosis).

The term "respiratory assistance" is generally known and encompasses the cardiopulmonary bypass (CPB), a technique that temporarily takes over the function of the heart and lungs during surgery, maintaining the circulation of blood and the oxygen content of the body. The CPB pump itself is often referred to as a heart-lung machine. Cardio-pulmonary bypass is commonly used in heart surgery because of the difficulty of operating on the beating heart.

The term "diagnosing" as used herein means assessing, determining, deciding, identifying, evaluating, or classifying whether an individual shows signs and symptoms of, suffers from and/or or developed a kidney injury in association with a surgical intervention or an acute invent at the time of isolation of the first (and/or second) sample for determination of the level of the biomarker of the invention such as L-FABP. As will be understood by those skilled in the art, such assessment is usually not intended to be correct for 100% of the individuals to be diagnosed. The term, however, requires that the assessment is correct for a statistically significant portion of the individuals (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably 0.1, 0.05, 0.01, 0.005, or 0.0001.

The term "associated with" as used herein encompasses a temporal, a statistical and/or a causal relationship between the kidney injury on the one hand and the surgical intervention or the acute event on the other hand.

The term "individual" as used herein relates to animals, preferably mammals, and, more preferably, humans, for example men or women. Preferably, the individual is an adult or a child. Preferably, the individual undergoing surgical intervention or who experienced an acute event does not suffer from a disease or condition prior to, at the time and/or around the time of the acute event or surgical intervention which is associated with increased L-FABP levels, preferably a disease or condition associated with a level of L-FABP of at least about 13 ng/ml, or more preferably at least about 15 ng/ml, most preferably about 17 ng/ml. Alternatively, a disease or condition associated with a level of L-FABP of at least about the 80th percentile, preferably at least about the 85th percentile, preferably at least about the 90th percentile, preferably at least about the 95th percentile, preferably at least about the 99th percentile of a healthy population, preferably derived from the respective ROC analysis.. Preferably, a disease or condition associated with increased L-FABP levels is a liver disease, a liver or kidney injury, cancer, or a chronic kidney injury. Additionally, the individual preferably shall not be requiring dialysis or having undergone dialysis within the 2 months prior to the acute event or the surgical intervention. Thus, the individual preferably does not suffer from a liver injury cancer, or a chronic kidney injury and the individual does not require dialysis or has undergone dialysis within 2 months prior to the acute event or the surgical intervention.

Apart from generally known solutions, methods and criteria for diagnosis of a liver injury of an individual may be based on determining the level of L-FABP or a liver enzyme (e.g. glutamate-dehydrogenase, ALT, GOT, gamma-GT) in a sample, isolated prior to an acute event or a surgical intervention. An elevation of the L-FABP level and/or liver enzymes in a sample isolated prior to an acute event or a surgical intervention compared to a reference level is indicative for a liver injury or liver disease.

Methods and criteria for diagnosing chronic kidney diseases are generally known in the art. One possibility to diagnose chronic kidney disease is detection of albumine excretion in urine >30 mg/24h.

In the context of the method for diagnosing a kidney injury in an individual after an acute event or after a surgical intervention, the term "reference level" as used herein, preferably, refers to a level which allows assessing whether an individual as referred to herein suffers from a kidney injury, preferably from acute kidney injury.

Preferably, the reference level is determined based on biomarker (like L-FABP) levels isolated from a reference individual or a group of reference individuals having undergone a surgical intervention and/or a reference individual or a group of reference individuals who (i) suffered from an acute event or underwent a surgical intervention and who (ii) were diagnosed as suffering from kidney injury after an acute event or after a surgical intervention.

Preferably, the sample from the reference individual or a group of reference individuals has been isolated during or after an acute event or a surgical intervention, preferably from a sample isolated within about 10 h after the acute event or after the surgical intervention. Preferably, the sample from the reference individual or a group of reference individuals has been isolated at the same point in time relative to the acute event or after the surgical intervention as the sample of the individual for whom the methods of the present inventions are carried out, e.g. if in the method of claim 1 the sample is isolated from the individual 5 hours after the surgical intervention the reference level is determined in a sample from the reference individual or a group of reference individuals isolated about 5h after a surgical intervention. Preferably, the sample from the reference individual or a group of reference individuals is isolated within the same time period or, more preferably, at essentially the same time point with respect to the acute event or the surgical intervention as the test sample from the test individual.

In a preferred embodiment of the invention, the reference level is determined from the second sample.

In another embodiment of the invention the reference level is based on a biomarker (like L-FABP) level obtained from a reference individual or a group of reference individuals having undergone a surgical intervention and/or a reference individual or a group of reference individuals who (i) suffered from an acute event or underwent a surgical intervention and who (ii) were diagnosed as not suffering from kidney injury after an acute event or after a surgical intervention. Moreover, the reference level may also be determined based on a sample known from an individual or a group of individuals known to be physiologically healthy,

The reference level applicable for an individual may vary depending on various physiological parameters such as age, gender, or subpopulation, as well as on the test format, the sample and the ligand used for the determination of the polypeptide or peptide referred to herein. These factors and ways to take them into account when determining the reference level are generally known in the field.

Reference levels can be calculated for a cohort of individuals as specified above based on the average or mean values for a given biomarker by applying standard statistically methods. In particular, accuracy of a test such as a method aiming to assess a condition, or not, is best described by its receiver-operating characteristics (ROC) (see especially Zweig 1993, Clin. Chem. 39: 561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate individuals to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the sum of number of true-positive plus number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the sum of number of true-negative plus number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has a ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively.

Accordingly, the reference level to be used for the aforementioned method of the present invention, i.e. a threshold which allows for discriminating between an individual suffering from kidney injury associated with an acute event or a surgical intervention and an individual not suffering from kidney injury associated with an acute event or a surgical intervention, is preferably established based on a ROC analysis for the individuals and cohort as described above. Depending on a desired sensitivity and specificity for a diagnostic method, the ROC plot allows deriving suitable thresholds. It will be understood that an optimal sensitivity is desired for excluding an individual not suffering from a kidney injury associated with an acute event or a surgical intervention (i.e. a rule out) whereas an optimal specificity is envisaged for an individual suffering from a kidney injury associated with an acute event or a surgical intervention (i.e. a rule in).

In the context of diagnosing a kidney injury in an individual after an acute event or after a surgical intervention a preferred reference level for L-FABP is at least about 13 ng/ml, more preferably at least about 15 ng/ml, most preferably about 17 ng/ml. Alternatively, the reference level corresponds to at least about the 80th percentile, preferably at least about the 85th percentile, preferably at least about the 90th percentile, preferably at least about the 95th percentile, preferably at least about the 99th percentile of a healthy population, the percentile may be of the specificity, e.g. derived from the respective ROC analysis

The term "comparing" as used herein encompasses comparing the level of the peptide or polypeptide comprised by the sample to be analysed with a level of a suitable reference level specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample or a ratio of amounts is compared to a reference ratio of amounts. The comparison referred to in step (b) of the methods of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format.

Preferably, a level of L-FABP in the sample from the individual which is at least equal or greater than the reference level supports, aids or justifies the diagnosis of a kidney injury associated with an acute event or a surgical intervention or that a kidney injury is very likely. Vice versa, a level of L-FABP in the sample from the individual which is below the reference level supports, aids or justifies the diagnosis that a kidney injury associated with an acute event or a surgical intervention can be ruled out or that the kidney injury is very unlikely.

It has been shown in the context of the present invention that the determination of L-FABP allows for an early assessment of kidney injury, i.e. even before kidney injury can be diagnosed via the determination of the aforementioned increase of serum creatinine. Accordingly, L-FABP is an earlier marker for kidney injury than creatinine.

The term "liver-type fatty acid binding protein" (L-FABP, frequently also referred to as FABP1 herein also referred to as liver fatty acid binding protein) relates to a polypeptide being a liver type fatty acid binding protein and to a variant thereof. Liver-type fatty acid binding protein is an intracellular carrier protein of free fatty acids that is expressed in the proximal tubules of the human kidney. For a sequence of human L-FABP, see e.g. Chan et al.: Human liver fatty acid binding protein cDNA and amino acid sequence, Functional and evolutionary implications, J. Biol. Chem. 260 (5), 2629-2632 (1985) or Gen-Bank Acc. Number M10617.1. The term "L-FABP" encompasses also variants of L-FABP, preferably, of human L-FABP. Such variants have at least the same essential biological and immunological properties as L-FABP, i.e. they bind free fatty acids and/or cholesterol and/or retinoids, and/or are involved in intracellular lipid transport. In particular, they share the same essential biological and immunological properties if they are detectable by the same specific assays referred to in this specification, e.g., by ELISA Assays using polyclonal or monoclonal antibodies specifically recognizing the L-FABP. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the human L-FABP, preferably over the entire length of the mature L-FABP protein sequence. Variants may be allelic variants or any other species specific homologs, paralogs, or orthologs. Moreover, the variants referred to herein include fragments of L-FABP or the aforementioned types of variants as long as these fragments have the essential immunological and biological properties as referred to above. Such fragments may be, e.g., degradation products of the L-FABP. Further included are variants which differ due to posttranslational modifications such as phosphorylation, glycosylation or myristylation. The term "L-FABP", preferably, does not include heart FABP, brain FABP and intestine FABP.

Preferably, the level of creatinine, NGAL, KIM-1, Cystatin C and adiponectin is additionally determined in a sample of the individual or in a second sample of the individual.

Creatinine as used herein is produced at a constant rate by muscle metabolism and is freely filtered by the glomeruli and also is secreted by the renal tubule. Because creatinine is secreted, creatinine clearance (Cr-Cl) overestimates GFR by about 10 to 20% in people with normal kidney function and up to 50% in those with advanced renal failure. Usually, creatinine is determined by enzymatic or colorimetric test systems in serum and urine. According to the definitions of AKI the level of creatinine has to be determined several days after the intervention or surgery for diagnosing an acute kidney injury.

The term "neutrophil gelatinase-associated Protein" (NGAL) as used herein refers to a protein having a molecular mass of 25 kDa in its glycosylated form and approximately 21 kDa in its deglycosylated form. It comprises 178 amino acids and has an amino acid sequence as described by Kjeldsen et al. in 1993 (Journal of Biological Chemistry, 268: 10425-10432). It is sometimes found as a heterodimer with human neutrophil gelatinase (matrix metalloproteinase 9), The expression of NGAL is known to be up-regulated in patients with acute renal dysfunction, especially after renal ischemic injury (Wagener et al,, 2006, Anesthesiology, 105: 485-491, The term "NGAL" as used herein encompasses also variants of NGAL, preferably, of human NGAL. Such variants have at least the same essential biological and immunological properties as NGAL, i.e, they prevent the degradation of matrix metalloproteinase 9. In particular, they share the same essential biological and immunological properties if they are detectable by the same specific assays referred to in this specification, e,g., by ELISA assays using polyclonal or monoclonal antibodies specifically recognizing the NGAL, Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the human NGAL. How to determine the degree of identity is well known to persons skilled in the art. Variants may be allelic variants or any other species specific homologs, paralogs, or orthologs. Moreover, the variants referred to herein include fragments of NGAL or the aforementioned types of variants as long as these fragments have the essential immunological and biological properties as referred to above. Such fragments maybe, e.g., degradation products of the NGAL. Further included are variants which differ due to posttranslational modifications such as phosphorylation, glycosylation, or myristylation.

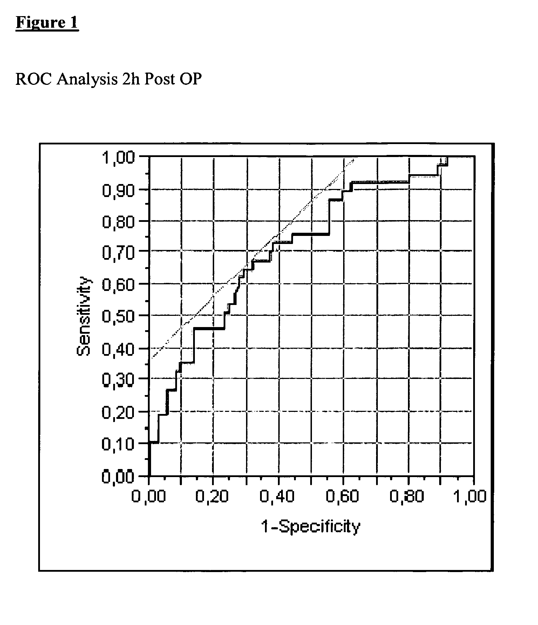

The term "kidney injury molecule-1" (KrM-1) as used herein relates to a type 1 membrane protein containing a unique six-cysteine Ig domain and a mucin domain in its extracellular portion. KIM-1 which is the sequence of rat 3-2 cDNA contains an open reading frame of 307 amino acids. The protein sequence of human cDNA clone 85 also contains one Ig, mucin, transmembrane, and cytoplasmic domain each as rat KIM-1. All six cysteines within the Ig domains of both proteins are conserved, Within the Ig domain, the rat Kim-1 and human cDNA clone 85 exhibit 68.3% similarity in the protein level. The mucin domain is longer, and the cyctoplasmic domain is shorter in clone 85 than rat KIM-1, with similarity of 49.3 and 34,8% respectively. Clone 85 is referred to as human SKIM-1 (for the structure of KIM-1 proteins see e.g. Ichimura et al., J Biol Cem, 273 (7), 4135-4142 (1998), in particular Fig. 1). Recombinant human KIM-1 exhibits no cross-reactivity or interference to recombinant rat- or mouse-KIM-1. KIM-1 mRNA and protein are expressed in high levels in regenerating proximal tubule epithelial cells which cells are known to repair and regenerate the damaged region in the postischemic kidney. KIM-1 is an epithelial cell adhesion molecule (CAM) up-regulated in the cells, which are dedifferentiated and undergoing replication after renal epithelial injury. A proteolytically processed domain of KIM-1 is easily detected in the urine soon after acute kidney injury (AKI) (Expert Opin. Med. Diagn. (2008) 2 (4): 387-398).KIM-1 referred to in accordance with the present invention further encompasses allelic and other variants of the specific sequence for human KIM-1 discussed above. Specifically, envisaged are variant polypeptides which are on the amino acid level preferably, at least 50%, 60%, 70%, 80%, 85%, 90%, 92%, 95%, 97%, 98%, or 99% identical, to human KIM-1. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity, Preferably, the default values of 5.00 for gap weight and 0.30 for gap weight length are used. Variants referred to above maybe allelic variants or any other species specific homologs, paralogs, or orthologs. Substantially similar and also envisaged are proteolytic degradation products which are still recognized by the diagnostic ligands directed against the respective full-length peptide. Also encompassed are variant polypeptides having amino acid deletions, substitutions, and/or additions compared to the amino acid sequence of human KIM-1 as long as the the polypeptides have KIM-1 properties.

The term "Cystatin C" as use herein is a small, 13 kDa, protein that is produced by virtually all nucleated cells. Its production rate is constant and is unaffected by inflammatory process, gender, age and muscle mass. In the normal kidney, Cystatin C is freely filtered at the globular membrane and then nearly completely reabsorbed and degraded by the proximal tubular cells. Therefore, the plasma concentration of Cystatin C is almost exclusively determined by the glomerular filtration rate (GFR), making Cystatin C an indicator of GFR. Cystatin C has advantages over routine clinical measures of renal function. It is more accurate than plasma creatinine, the Cockcroft-Gault estimation of creatinine clearance and is more reliable than the 24-h creatinine clearance. Cystatin C has been shown to increase earlier than serum creatinine in patients developing AKI one to two days earlier than serum creatinine.

Cystatin C as used used herein encompasses also variants of Cystatin C, preferably, of human Cystatin C, Such variants have at least the same essential biological and immunological properties as Cystatin C.In particular, they share the same essential biological and immunological properties if they are detectable by the same specific assays referred to in this specification, e.g., by ELISA assays using polyclonal or monoclonal antibodies specifically recognizing the Cystatin C. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the human Cystatin C. How to determine the degree of identity is well known to persons skilled in the art. Variants may be allelic variants or any other species specific homologs, paralogs, or orthologs. Moreover, the variants referred to herein include fragments of Cystatin C or the aforementioned types of variants as long as these fragments have the essential immunological and biological properties as referred to above. Such fragments may be, e.g., degradation products of the Cystatin C. Further included are variants which differ due to posttranslational modifications such as phosphorylation, glycosylation, or myristylation.

Adiponectin as used herein is a polypeptide (one of several known adipocytokines) secreted by the adipocyte. In the art, adiponectin is frequently also referred to as Acrp30 and apM1. Adiponectin has recently been shown to have various activities such as antiinflammatory, antiatherogenic, preventive for metabolic syndrome, and insulin sensitizing activities. Adiponectin is encoded by a single gene, and has 244 amino acids, its molecular weight is approximately 30 kDa. The mature human adiponectin protein encompasses amino acids 19 to 244 of full-length adiponectin. A globular domain is thought to encompass amino acids 107-244 of full-length adiponectin. The sequence of the adiponectin polypeptide as used herein is well know in the art, and, e.g., disclosed in WO/2008/084003.

Adiponectin as used herein, preferably, relates to total adiponectin, which encompasses low molecular weight adiponectin, mid molecular weight adiponectin and high molecular weight adiponectin. The terms high molecular weight adiponectin, low and mid molecular weight adiponectin and total adiponectin are understood by the skilled person. Preferably, the adiponectin is human adiponectin. Methods for the determination of adiponectin are, e.g., disclosed in US 2007/0042424 A1 as well as in WO/2008/084003. The amount of adiponectin may be determined in a urine sample.

The adiponectin referred to in accordance with the present invention further encompasses allelic and other variants of the specific sequence for human adiponectin discussed above. Specifically, envisaged are variant polypeptides which are on the amino acid level preferably, at least 50%, 60%, 70%, 80%, 85%, 90%, 92%, 95%, 97%, 98%, or 99% identical, to human adiponectin. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85; 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic ligands directed against the respective full-length peptide. Also encompassed are variant polypeptides having amino acid deletions, substitutions, and/or additions compared to the amino acid sequence of human adiponectin as long as the polypeptides have adiponectin properties, in particular Insulin sensitizing properties.

Preferably, an increased level and, more preferably, a significantly increased level, of L-FABP in the first sample compared to the level of L-FABP in the second sample is indicative for the diagnosis of a kidney injury (or risk thereof) in the individual who had an acute event or underwent surgical intervention.

Preferably, additionally to L-FABP, a significantly increased level of a marker selected from creatinine, NGAL, KIM-1, Cystatin C and adiponectin, in the first sample is compared to the respective reference level and the increased marker level and the increased L-FABP level is indicative for the diagnosis of a kidney injury (or risk thereof) associated with an acute event or a surgical intervention in the individual.

Preferably, an essentially identical level of L-FABP and or an increased level of L-FABP and at least one marker selected from creatinine, NGAL, KIM-1, Cystatin C and adiponectin in the sample from the test individual (or in the samples from the test individual in case L-FABP and are determined in two different samples) relative to the respective reference level is indicative for the diagnosis (or for the prediction of a risk) of a kidney injury associated with an acute event or a surgical intervention.

The term "sample" refers to a sample of a body fluid selected from blood, i.e. whole blood, plasma, or serum, or urine, or to a sample of separated cells or to a sample from a tissue or an organ. Samples of body fluids can be isolated by well-known techniques. Tissue or organ samples may be isolated from any tissue or organ by, e.g., biopsy. Separated cells may be isolated from the body fluids or the tissues or organs by separating techniques such as centrifugation or cell sorting. Preferably, cell-, tissue- or organ samples are isolated from those cells, tissues or organs which express or produce the peptides referred to herein.

The level of the markers determined in the context of the present invention (i.e. L-FABP, or a variant thereof and optionally, creatinine, NGAL, KIM-1, Cystatin C and adiponectin or a variant thereof will preferably be determined in a whole blood sample, plasma and serum of the respective individual. As the case may be, the marker(s) other than L-FABP may also be determined in a urine sample of the respective subject.

In a preferred embodiment of the present invention, in case of the surgical intervention, the L-FABP level is additionally determined in a second blood sample isolated before the first sample, preferably isolated before or during the surgical intervention or the acute event, and an increase of the L-FABP level in the first blood sample relative to the second blood sample is indicative of a kidney injury.

The "first sample" according to the present invention is, preferably, isolated after an acute event or after a surgical intervention, i.e. after the individual undergoes the surgical procedure. At least one further sample (e.g. a third sample) may be isolated in order to further monitor the change of the level of L-FABP to herein. Such further samples may be isolated, preferably, 1 to 10 hours, 1 to 8 hours, and, more preferably, 2 to 4 hours after the first sample. Optionally, a further sample is isolated 1 or 2 or 3 or 4 days after the first sample.

The "second sample" may be a sample which is isolated in order to monitor a change of the level of the L-FABP as compared to the level of the respective marker in the first sample. Preferably, the second blood sample isolated before or simultaneously with the first sample. The second sample may be isolated before or during the surgical intervention or the acute event. It is particularly contemplated that the second sample has been isolated before the surgical procedure or before the acute event has been completed.

Preferably, an increased level, more preferably, a statistically significant increase of the level of L-FABP in the first sample as compared to the level of L-FABP in the second sample is indicative for the diagnosis of a kidney injury or of the risk of an individual to suffer from a kidney injury after an acute event or after a surgical intervention in the future. As used herein, the comparison of the level of L-FABP in the first sample relative to the second sample can be made by subtracting or dividing (ratio calculation) of the respective levels. Preferably the increase is determined by calculating the ratio of the level of L-FABP in the first sample compared to the level in the second sample.

In a preferred embodiment, an increase of the level of L-FABP in the first sample compared to the level in the second sample which is considered to be statistically significant is an increase of at least about factor 2.5, more preferable of at least about factor 3, most preferable of at least about factor 3.5. Alternatively, the increase corresponds to a specificity determined from the respective ROC analysis of possible L-FABP ratios of the first and second sample of at least about 80%, more preferable of at least about 90%, or most preferable of at least about 95%.

The statistical increase of being indicative for kidney injury may depend on the time point when the first sample is isolated and the timing of the isolation of the first sample versus the second sample. A skilled artisan is well able to determine such statistically significant increases under varying time points and intervals of sampling. A preferred time interval between the isolation of the first sample and the second sample is at least about 1h, at least about 2h, at least about 3h, at least about 4h, at least about 6h, at least about 8h.

As the case may be, the statistical increase of being indicative for kidney injury may depend on the time point when the first sample is isolated and the timing of the isolation of the first sample versus the acute event or the surgical intervention. A skilled artisan is well able to determine such statistically significant increases under varying time points and intervals of sampling. A preferred time interval between the isolation of the first sample and the acute event or the surgical intervention is at least about 1h, at least about 2h, at least about 3h, at least about 4h, at least about 6h, at least about 8h.

As set forth above, the individual according to the present invention, preferably, has undergone or shall undergo a surgical intervention or experienced an acute event at the time at which the sample as referred to in the context of the aforementioned method is isolated.

The term "after the acute event or after the surgical intervention", preferably relates to isolating the sample within about 0.5 hours, within about 1 hour, within about 2 hour, within about 4 hours, within about 6 hours, or within about 8 hours after the acute event or after the surgical intervention.

In a preferred embodiment of the present invention the first blood sample is isolated within about 10 min, within about 20 min, within about 30 min, within about 40 min, within about 50 min, within about 1h, within about 2 h, within about 4 h, within about 6 h or within about 8 h after the acute event or after the surgical intervention, In particular, the first blood sample is isolated within about 10 h after the acute event or after the surgical intervention.

In the context of the present invention and the phrase "isolating a sample within about 10 h after the surgical intervention" the time "after surgical intervention" may be calculated from the time (i) the individual enters the room where the surgical intervention is carried out, (ii) the time the first physician carries out an anamnesis of the individual, (iii) the surgical intervention is initiated, e,g. when the anaesthesia is administered, (iv) the surgical intervention has been completed, (v) preferably, when the respiratory assistance is shut-off, (vi) the individual is transferred from the room where the surgical intervention is carried out, (vii) the arrival of the individual at the intensive care unit after completion of the surgical intervention, (vii) the arrival of the individual at the sick room, or (viii) discharge of the individual from the institution, office or hospital where the surgical intervention is carried out.

In a preferred embodiment of the invention, the level of at least one biomarker - preferably the biomarker is L-FABP and optionally an additional marker mentioned above - is determined in a second sample that has been isolated prior to the first sample. If the level of at least one biomarker in the first sample is equal to or greater than the level in the second sample this is indicative of kidney injury and preferably also vice versa, i.e. if the level of the least one biomarker in the first sample is equal to or below the level in the second sample this is indicative that the individual does not suffer from kidney injury.

Preferably, the period of time between isolating the first sample and the second sample as well as the time points for isolating the samples in the test individual corresponds to the period of time between isolating the first sample and the second sample to the time points for isolating the sample(s) in the control individual(s) used for the calculation of the reference level.

As the case may be, the period of time between isolating the first sample and the acute event or the surgical intervention as well as the time points for isolating the samples in the test individual corresponds to the period of time between isolating the first sample and the acute event or the surgical intervention to the time points for isolating the sample(s) in the control individual(s) used for the calculation of the reference level. Determining the level of a peptide or polypeptide referred to in this specification relates to measuring the level or concentration, preferably, semi-quantitatively or quantitatively. Measuring can be done directly or indirectly. Direct measuring relates to measuring the level or concentration of the peptide or polypeptide based on a signal which is obtained from the peptide or polypeptide itself and the intensity of which directly correlates with the number of molecules of the peptide present in the sample. Such a signal-sometimes referred to herein as intensity signal -may be obtained, e,g., by measuring an intensity value of a specific physical or chemical property of the peptide or polypeptide. Indirect measuring includes measuring of a signal obtained from a secondary component (i,e, a component not being the peptide or polypeptide itself) or a biological read out system, e.g., measurable cellular responses, ligands, labels, or enzymatic reaction products.

In accordance with the present invention, determining the level of a peptide or polypeptide can be achieved by all known means for determining the level of a peptide in a sample. The means comprise immunoassay and methods which may utilize labelled molecules in various sandwich, competition, or other assay formats, Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the level of polypeptide present in a sample. Further suitable methods comprise measuring a physical or chemical property specific for the peptide or polypeptide such as its precise molecular mass or NMR spectrum. The methods comprise, preferably, biosensors, optical devices coupled to immunoassays, biochips, analytical devices such as mass-spectrometers, NMR- analyzers, or chromatography devices, Further, methods include micro-plate ELISA-based methods, fully-automated or robotic immunoassays (available for example on ElecsysTM analyzers), CUBA (an enzymatic Cobalt Binding Assay, available for example on Roche-HitachiTM analyzers), and latex agglutination assays (available for example on Roche-HitachiTM analyzers).

Preferably, determining the level of a peptide or polypeptide comprises the steps of (a) contacting a cell capable of eliciting a cellular response the intensity of which is indicative of the level of the peptide or polypeptide with the peptide or polypeptide for an adequate period of time, (b) measuring the cellular response. For measuring cellular responses, the sample or processed sample is, preferably, added to a cell culture and an internal or external cellular response is measured, The cellular response may include the measurable expression of a reporter gene or the secretion of a substance, e.g. a peptide, polypeptide, or a small molecule. The expression or substance shall generate an intensity signal which correlates to the level of the peptide or polypeptide,

Also preferably, determining the level of a peptide or polypeptide comprises the step of measuring a specific intensity signal obtainable from the peptide or polypeptide in the sample. As described above, such a signal may be the signal intensity observed at an m/z variable specific for the peptide or polypeptides observed in mass spectra or a NMR spectrum specific for the peptide or polypeptide.

Determining the level of a peptide or polypeptide may, preferably, comprises the steps of (a) contacting the peptide with a specific ligand, (b) (optionally) removing non-bound ligand, (c) measuring the level of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. A preferred ligand is a ligand binding to L-FABP or to a marker selected from creatinine, NGAL, KIM-1, Cystatin C and adiponectin.

Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well, Such hybrids can be prepared by several methods well known in the art. Preferably, the ligand or agent binds specifically to the peptide or polypeptide, preferably to L-FABP or a marker selected from creatinine, NGAL, KIM-1, Cystatin C and adiponectin. Specific binding according to the present invention means that the ligand or agent should not bind substantially to ("cross-react" with) another peptide, polypeptide or substance present in the sample to be analysed like I-FABP or H-FABP. Preferably, the specifically bound peptide or polypeptide should be bound with at least 3 times higher, more preferably at least 10 times higher and even more preferably at least 50 times higher affinity than any other relevant peptide or polypeptide, Non-specific binding may be tolerable, if it can still be distinguished and measured unequivocally, e.g. according to its size on a Western Blot, or by its relatively higher abundance in the sample. Binding of the ligand can be measured by any method known in the art. Preferably, the method is semiquantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the level of a protease can be measured by measuring the level of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the level of substrate is saturating. The substrate may also be labelled with a detectable label prior to the reaction. Preferably, the sample is contacted with the substrate for an adequate period of time. An adequate period of time refers to the time necessary for a detectable, preferably measurable, level of product to be produced. Instead of measuring the level of product, the time necessary for appearance of a given (e.g. detectable) level of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. The secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidinbiotin system (Vector Laboratories, Inc.). The ligand or substrate may also be "tagged" with one or more tags as known in the art Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star$^{TM}$ (Amersham Biosciences), ECF$^{TM}$ (Amersham Biosciences). A suitable enzyme-substrate combination may result in a coloured reaction product, fluorescence or chemoluminescence, which can be measured according to methods known in the art (e.g, using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I 32P, 33P, 14C, 3H and the like. A radioactive label can be detected by any method known and appropriate, e.g, a light-sensitive film or a phosphor imager or by scintillation counting. Suitable measurement methods according the present invention also include precipitation (particularly immunoprecipitation), electrochemiluminescence (electrogenerated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), FCM (Flow cytrometry), FRET (Flourescence-Resonance Energy Transfer), -turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The level of a peptide or polypeptide may be, also preferably, determined as follows: (a) contacting a solid support comprising a ligand for the peptide or polypeptide as specified above with a sample comprising the peptide or polypeptide and (b) measuring the level peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc, The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing the ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "level" as used herein encompasses the absolute amount of a polypeptide or peptide in the sample, the relative level like a concentration of the polypeptide or peptide in the sample as well as any value or parameter which correlates thereto or can be derived therefrom. Such values or parameters comprise intensity signal values from all specific physical or chemical properties obtained from the peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned levels or parameters can also be obtained by all standard mathematical operations.

The expression "comparing the level in the first sample to the level in the second sample" as used herein in the context of the present invention encompasses comparing the level of a L-FABP in a first sample with a level of the marker in a second sample. The terms "first sample" and "second sample" are specified herein above.

The term "comparing" as used herein refers to a comparison of corresponding parameters or values, e.g., an absolute level is compared to an absolute reference level while a concentration is compared to a reference concentration or an intensity signal obtained from a test sample is compared to the same type of intensity signal of a reference sample, The comparison referred to in step (b) of the methods of the present invention may be carried out manually or computer assisted. For a computer assisted comparison, the value of the determined level may be compared to values corresponding to suitable references which are stored in a database by a computer program. The computer program may further evaluate the result of the comparison, i.e. automatically provide the desired assessment in a suitable output format. Based on the comparison of the level determined in step a) and the reference level, it is possible to assess whether an individual suffers from kidney injury associated with the acute event or the surgical intervention. Therefore, the reference level is to be chosen so that either a difference or a similarity in the compared levels allows for diagnosing kidney injury, and, thus, for identifying those individuals which suffer from kidney injury associated with the acute event or the surgical intervention (rule-in) or not (rule out). Likewise, in the method of predicting the risk of an individual to suffer from a kidney injury, based on the comparison of the level determined in step a) and the reference level, it is possible to predict whether an individual has a risk to suffer from kidney injury associated with the acute event or the surgical intervention in the future. Therefore, the reference level is to be chosen so that either a difference or a similarity in the compared levels allows for predicting the risk, and, thus, for identifying those individuals which have a significant risk to suffer from kidney injury (rule-in) or do not have a significant risk (rule out).

As set forth above, in a preferred embodiment the diagnosis of kidney injury is, preferably, based on the comparison of the level of L-FABP in a first sample to the level of the L-FABP in a second sample isolated before the first sample. Such serial sample measurement based diagnosis may for example be useful in an individual displaying levels of the markers (e.g. L-FABP) above the level characteristic for a healthy individual but below the above referenced reference level indicative of a kidney injury.

In the context of the present inventions the term "about" as used herein refers to +/-20%, preferably +/-10%, preferably, +/- 5% of a given measurement or value.

In a second aspect of the present invention it is provided a method of predicting the risk of an individual to suffer from a kidney injury after an acute event or after a surgical intervention in the future comprising the steps of:

a) determining the level of L-FABP or a variant thereof in a first blood sample isolated from an individual, wherein the sample is isolated within 10 h after the event or after the surgical intervention; and
b) comparing the level determined in step a) with a reference level, wherein a level of L-FABP equal to or greater than the reference level is indicative of a risk of an individual to suffer from a kidney injury.

Preferably the method further comprises a step of predicting the risk of the individual based on the comparison of step b).

Unless stated differently the explanations and definitions provided above apply mutatis mutandis to the method of predicting the risk of an individual to suffer from a kidney injury after an acute event or after a surgical intervention in the future,

The expression "predicting the risk of an individual to suffer from a kidney injury after an acute event or after a surgical intervention in the future" as used herein refers to assessing the probability of an individual to suffer from a kidney injury in the future, i.e. within the predictive window after the sample(s) were isolated which sample(s) form the basis for the assessment. In accordance with the present invention, the predictive time window, i.e. the meaning of the term "future", preferably, is about 1 h, up to about 2 h, up to about 4 h, up to about 6 h, up to about 8 h, up to about 10 h, up to about 12 hours, up to about 1 day, up to about 2 days, up to about 3 days, up to about 4 days, up to about 5 days, up to about 6 days or up to about 1 week after the surgical intervention or after the acute event, as the case maybe. The term, preferably, relates to predicting whether or not there is an increased risk for a kidney injury compared to the average risk for developing a kidney injury in a population of individuals rather than giving a precise probability for the risk

In the context of the present invention the term "after the surgical intervention" the time "after surgical intervention" may be calculated from the time (i) the individual enters the room where the surgical intervention is carried out, (ii) the time the first physician carries out an anamnesis of the individual, (iii) the surgical intervention is initiated, e.g. when the anaesthesia is administered, (iv) the surgical intervention has been completed, (v) the individual is transferred from the room where the surgical intervention is carried out, (vi) the arrival of the individual at the intensive care unit after completion of the surgical intervention, (vii) the arrival of the individual at the sick room, or (viii) discharge of the individual from the institution, office or hospital where the surgical intervention is carried out, (ix) and most preferably, the respiratory assistance of the individual is shut-off

In the context of the method for predicting the risk of an individual to suffer from a kidney injury, the term "reference level" as used herein, preferably, refers to a level which allows predicting the risk of an individual to suffer from a kidney injury after an acute event or after a surgical intervention in the future. Preferably, the reference level is determined based on a biomarker (preferably L-FABP) level(s) isolated from at least one reference individual or from a reference population who

- had an acute event or underwent a surgical intervention and who developed a kidney injury within the predictive time window, or
- had an acute event or underwent a surgical intervention and who did not develop a kidney injury within the predictive time window, or
- did not have an acute event or did not undergo a surgical intervention and who did not develop a kidney injury within the predictive time window.

Preferably, the sample from the reference individual or group of reference individuals has been isolated during or after an acute event or during or after a surgical intervention, preferably from a sample isolated within about 10 h after the acute event or after the surgical intervention, as the case maybe. Preferably, the sample from the reference individual (or group of reference individuals) from which the reference level is derived is isolated within the same time period or, more preferably, at essentially the same time point with respect to the acute event or the surgical intervention as the test sample from the test individual. Moreover, the reference level may also be determined based on a sample known from an individual or a group of individuals known to be physiologically healthy.

In another embodiment of the present invention, additionally the L-FABP level is determined in a second blood sample isolated before the first sample, preferably before or during the surgical intervention. A level of L-FABP level in the first blood sample of equal to or greater than the second blood sample is indicative of a (significant or high) risk to suffer from kidney injury. Vice versa, a level of L-FABP level in the first blood below the second blood sample is indicative of the individual not to have a (low or neglectable) risk to suffer from kidney injury.

In general, for determining the respective level(s) or level ratio(s) allowing to establish the desired prediction in accordance with the respective embodiment of the present invention, ("threshold", "reference level"), the level(s) or level ratio(s) of the respective peptide or peptides are determined in appropriate reference individual (s) defined above. The results which are obtained are collected and analyzed by statistical methods known to the person skilled in the art. The obtained threshold values are then established in accordance with the desired probability of suffering from the disease in the future and linked to the particular threshold value. For example, it may be useful to choose the median value, the 60th, 70th, 80th, 90th, 95th or even the 99th percentile of the healthy and/or non-healthy individual collective, in order to establish the threshold value(s), reference level(s).

In order to test if a chosen reference level yields a sufficiently safe prediction of patients being at risk to suffer from kidney injury in the future, one may for example determine the efficiency (E) of the methods of the invention for a given reference value using the following formula:

$$E = (TP / TO) \times 100;$$

wherein TP = true positives and TO = total number of tests = TP + FP + FN + TN, wherein FP = false positives; FN = false negatives and TN = true negatives. E has the following range of values: 0 < E < 100). Preferably, a tested reference level yields a sufficiently safe diagnosis provided the value of E is at least about 50, more preferably at least about 60, more preferably at least about 70, more preferably at least about 80, more preferably at least about 90, more preferably at least about 95, more preferably at least about 98.

In the context of the method of prediction invention, a preferred reference levels for L-FABP indicative of the individual to be at risk to suffer from kidney injury, preferably AKI, in the future is at least about 13 ng/ml, or more preferably at least about 15 ng/ml, most preferably about 17 ng/ml. Alternatively, the reference level corresponds to at least about the 80th percentile, preferably at least about the 85th percentile, preferably at least about the 90th percentile, preferably at least about the 95th percentile, preferably at least about the 99th percentile of a reference population, the percentile may be of the specificity, e.g. derived from the respective ROC analysis.

Preferably, a determined level of L-FABP-level above the reference level is indicative of an elevated or high risk to suffer from kidney injury in the future. For example, elevated or high risk may be the a probability of at least about 50%, at least about 60%, at least about 70%, at least about 80%, at least about 90%, at least about 95%, at least about 99%, that the individual will develop a kidney injury in the future (i.e. within the predictive time window).

In another embodiment, additionally the level of a marker selected from creatinine, NGAL, KIM-1, Cystatin C and adiponectin is determined in the first and/or in a second sample isolated from the individual.

Preferably, an increased level and, more preferably, a significantly increased level, of L-FABP in the first sample as compared to the level of L-FABP in the second sample concomitant with an increased level and, more preferably, a significantly increased level of a marker selected from creatinine, NGAL, KIM-1 , Cystatin C and adiponectin in another sample as compared to the level of L-FABP in the first sample is indicative for the diagnosis of a kidney injury (or risk thereof) associated with an acute event or a surgical intervention in the individual.

In another embodiment of the invention, the method further comprises the step of selecting or adapting a renal therapy for an individual suffering from or having a risk to suffer from kidney injury after an acute event or after a surgical intervention.

It is to be understood that the definitions and explanations of the terms made above and below apply mutatis mutandis for all embodiments described in this specification and the accompanying claims (except stated otherwise),

In a third aspect of the invention it is provided method for selecting a renal therapy for an individual suffering from or having a risk to suffer from kidney injury after an acute event or after a surgical intervention comprising the steps of:

a) determining the level of L-FABP or a variant thereof in a first blood sample of an individual isolated within 10 h after the event or after the surgical intervention;
and

b) comparing the level determined in step a) with a reference level;
wherein the renal therapy is selected based on the comparison of step b).

The renal therapy encompasses among others the administration or avoidance of nephrotoxic medication or treatment.
Furthermore, it is also envisaged to monitor the blood pressure once the diagnosis of kidney injury has been made and/or the intake of fluid. In case of increased blood pressure, blood pressure lowering medicaments shall be administered, or in case of decreased blood pressure, blood pressure increasing methods or drugs shall be applied. Moreover, careful fluid balance is important in an individual for which the diagnosis of kidney injury has been established.

In a preferred embodiment of the present invention, in patients diagnosed as having kidney injury or as having a risk to suffer from kidney injury in the future, the renal therapy is adapted by reducing or discontinuing the administration of nephrotoxic medication or treatment, by increasing, reducing or discontinuing the intake or output of fluid, or by adapting the treatment so as to reduce or increase the blood pressure.

In a preferred embodiment of the method of the present invention, the method further comprises the step of recommending a therapy.

The term "recommending" as used herein means establishing a proposal for a therapy which could be applied to the individual. However, it is to be understood that applying the actual therapy whatsoever is preferably not comprised by the term. The therapy to be recommended depends on the outcome of the diagnosis provided by the method of the present invention. Preferably, nephrotoxic medication shall be avoided once the diagnosis or prediction of kidney injury has been made. In particular, it is also envisaged to monitor the blood pressure once the diagnosis or prediction of kidney injury has been made. In case of increased blood pressure, blood pressure lowing medicaments shall be administered, or in case of decreased blood pressure, blood pressure increasing methods or drugs shall be applied.

Moreover, the present invention envisages a kit adapted for carrying out the method of the present inventions, the device comprising a binding ligand for the biomarker L-FABP. Moreover, the kit may further comprise the biomarker L-FABP and/or instructions for carrying out the method. The kit may further comprise a device for quantitative determination of L-FABP.

The term "kit" as used herein refers to a collection of the aforementioned components, preferably, provided separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standards, reference samples and control samples.

Another embodiment of the present invention is an in vitro use of an antibody binding L-FABP or a variant thereof for diagnosing a kidney injury in an individual after an acute event or after a surgical intervention or for predicting the risk of an individual to suffer from a kidney injury after an acute event or after a surgical intervention in the future, in a sample of an individual, wherein level of L-FABP is detected in a blood sample isolated within about 10 h after the acute event or after the surgical intervention.

A further embodiment of the present invention is an in vitro use of an antibody binding L-FABP or a variant thereof for selecting, deciding or adapting a renal therapy for an individual suffering from a kidney injury after an acute event or after a surgical intervention, being associated with a risk to suffer from a kidney injury after an acute event or after a surgical intervention in the future, wherein the level of L-FABP is detected in a blood sample isolated within about 10 h after the acute event or after the surgical intervention.

Moreover, the present invention relates to a device adapted for carrying out the method of the present invention for diagnosing or predicting kidney injury associated with an acute event or a surgical intervention comprising

a. an analyzing unit comprising a binding ligand which specifically binds to L-FABP, the unit being adapted for determining the level of L-FABP in a first and a second sample from an individual; and

b. an evaluation unit for comparing the determined level in the first sample with the level in the second sample whereby AKI associated with an acute event or a surgical intervention can be diagnosed, the unit comprising a database with ratios of the level of L-FABP in the first sample as compared to the second sample, the ratios being, preferably, derived from an individual or a group of individuals known to have developed kidney injury associated with an acute event or the surgical intervention or more preferably derived from an individual or a group of individuals known not to have developed kidney injury associated with an acute event or the surgical intervention and a computer-implemented algorithm for carrying out a comparison step,

It is to be understood that the definitions and explanations of the terms made above and below apply mutatis mutandis for all embodiments described in this specification and the accompanying claims (except stated otherwise).

In the context of the aforementioned device, the reference levels are preferably derived from a sample of (reference) individuals as defined above,

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises the detection agents in immobilized form on a solid support which is to be contacted to the sample comprising the biomarkers the level of which is to be determined. Moreover, the analyzing unit can also comprise a detector which determines the level of binding ligand which is specifically bound to the biomarker(s). The determined level can be transmitted to the evaluation unit. The evaluation unit comprises a data processing element, such as a computer, with an implemented algorithm for carrying out a comparison between the determined level and a suitable reference (e.g. a reference level, or the level of the marker in a first or second sample from the individual). Suitable references can be derived from samples of individuals to be used for the generation of reference levels as described elsewhere herein above. The results may be given as output of parametric diagnostic raw data, preferably, as absolute or relative levels. It is to be understood that these data will need interpretation by the clinician. However, also envisaged are expert system devices wherein the output comprises processed diagnostic raw data the interpretation of which does not require a specialized clinician.

All references cited in this specification are herewith incorporated by reference with respect to their entire disclosure content and the disclosure content specifically mentioned in this specification.

## BRIEF DESCRIPTION OF THE DRAWINGS

Figure 1:

ROC analysis for determining the level of L-FABP in a blood sample isolated from two groups of individuals, one which did and one which did not develop AKI after cardiac surgery, wherein the samples were isolated 2 h after the surgical intervention.

Figure 2:

ROC analysis for determining the level of L-FABP in a blood sample isolated from two groups of individuals, one which did and one which did not develop AKI after cardiac surgery, wherein the samples were isolated 4 h after the surgical intervention.

Figure 3:

ROC analysis for determining the level of L-FABP in a blood sample isolated from two groups of individuals, one which did and one which did not develop AKI after cardiac surgery,, wherein the samples were isolated 24 h after the surgical intervention.

Figure4:

Comparison of L-FABP levels in plasma or serum. From 32 persons blood has been taken at the same timepoint in two separate tubes, processed to serum or EDTA-plasma, respectively, and L-FABP tested. Pearson correlation was r=0.999. The solid line in the graph represents the bisector of the angle.

Figure 5:

Comparison of L-FABP in plasma after cardiac surgery in patients suffering from AKI or not suffering from AKI (No AKI). The end of the box shows the 25th or 75th percentile, respectively, and the whiskers are placed to the 5th or 95th percentile. The line within the box shows the median. The levels of significance of the differences between the groups are calculated using the Wilcoxon test.

The following example is only intended to illustrate the present invention. It shall not limit the scope of the invention in any way.

**Example** 1

**Patients, Materials and Methods**

Study description

107 patients underwent cardiac surgery (bypass or valve replacement). From all patients blood samples were taken the day before surgery (pre-OP) and 2h, 4h, 24h, 3 days and in some cases 7 days after surgery. Time point 0 was defined as the shut-down of the respiratory assistance (i.e. the heart-lung machine). After centrifugation EDTA-plasma or serum was stored at -80°C until testing,

Inclusion criteria: cardiac surgery using a heart-lung machine, sample from the day before surgery present, informed consent, >18 years of age.

Exclusion criteria: no informed consent, no sample from the day before surgery, <18 years, pregnancy.

Sample determination

A kit from R&D Systems, Catalogue No. Z-001 was used for quantitative determination of L-FABP in plasma samples, The assay uses two L-FABP-specific mouse monoclonal antibodies forming a sandwich assay, described in Kamijo, A, et al., J Lab Clin Med 2004, 143, 23-30. The generation of monoclonal antibodies specific for hL-FABP is described in Kamijo, A. et al., AJP October 2004, Vol. 165, No. 4,1243-1255,

Analysis

Patients were diagnosed retrospectively to suffer from AKI when their serum creatinine level were raised at least 50% or 0.3 mg/dL within 3 days after surgery (AKIN definition), compared to the individual's pre-surgery level.

Differences of biomarker levels between the groups of AKI and non-AKI at the 5 time points mentioned above were analyzed using the nonparametric Wilcoxon test Sensitivities and specificities were calculated with the ROC analysis (receiver operated curve fit).

Results

In 73 patients out of the 107 patients who underwent cardiac surgery no AKI was detected, whereas 34 patients developed AKI within 3 days using the AKIN criteria based on serum creatinine.

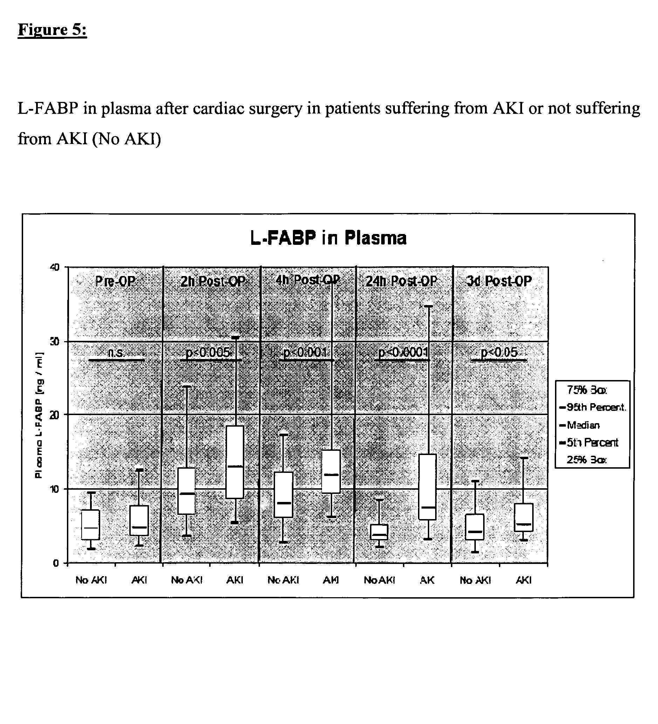

L-FABP from plasma samples was analyzed for its suitability to diagnose AKI before it was evident using creatinine. It is evident from figure 5 that plasma-L-FABP based detection allows for a robust and very early diagnosis of AKI as early as 2 hours post-surgery (post-OP). Notably, in the current study plasma L-FABP permits early identification of a subgroup of patients at increased risk of acute kidney injury well before established kidney functions tests, e.g, based on the determination of creatinine. This early identification was not possible before the present invention.

L-FABP in AKI diagnosed individuals was already significantly elevated in plasma relative to non-AKI individuals as early as 2h after surgery and beyond that timepoint.

**Claims**

1. A method for diagnosing a kidney injury in an individual after an acute event or after a surgical intervention comprising the steps of:

   a) determining the level of liver-type fatty acid binding protein (L-FABP) or a variant thereof in a first blood sample isolated from an individual, wherein the sample is isolated within about 10 h after the acute event or after the surgical intervention; and
b) comparing the level determined in step a) with a reference level;
wherein a level of L-FABP or a variant thereof which is at least equal to or greater than the reference level is indicative of kidney injury.

2. The method according to claim 1, wherein the acute event is selected from an accident, a burn, and a trauma.

3. The method according to any one of claims 1 to 2, wherein the surgical intervention is selected from a cardiac surgery, a cardio-pulmonary surgery, and a surgery associated with the use of respiration support.

4. The method according to any one of claims 1 to 3, wherein the reference level of L-FABP is at least about 13 ng/ml or a level of L-FABP corresponding to at least about 80% percentile of a healthy population.

5. The method according to any one of claims 1 to 4, wherein additionally the level of a marker selected from creatinine, NGAL, KIM-1, Cystatin C and adiponectin is determined in the first and/or in a second sample isolated from the individual.

6. The method according to any one of claims 1 to 5, wherein the blood sample is selected from whole blood, plasma and serum.

7. The method according to any one of claims 1 to 6, wherein in case of the surgical intervention additionally the L-FABP level is determined in a second blood sample isolated before the first sample, preferably isolated before or during the surgical intervention or the acute event, and an increase of the L-FABP levels of the first compared to the L-FABP level of the second blood sample of at least a factor of about 2,5 is indicative of a kidney injury.

8. The method according to any one of claims 1 to 7, wherein the first blood sample is isolated within an interval selected from about 2h, about 4 h, about 6 h, about 8 h, and about 10h after the acute event or after the surgical intervention.

9. A method of predicting the risk of an individual to suffer from a kidney injury after an acute event or after a surgical intervention in the future, comprising the steps of:

a) determining the level of L-FABP or a variant thereof in a first blood sample isolated from an individual, wherein the sample is isolated within 10 h after the event or after the surgical intervention; and
b) comparing the level determined in step a) with a reference level,
wherein a level of L-FABP equal to or greater than the reference level is indicative of a risk of an individual to suffer from a kidney injury.

10. The method according to claim 9, wherein the reference level of L-FABP is at least about 13 ng/ml or a level of L-FABP corresponding to at least about 80% percentile of a reference population.

11. A method for selecting a renal therapy for an individual suffering from or having a risk to suffer from kidney injury after an acute event or after a surgical intervention comprising the steps of:

a) determining the level of L-FABP or a variant thereof in a first blood sample of an individual isolated within 10 h after the event or after the surgical intervention; and
b) comparing the level determined in step a) with a reference level;
wherein the renal therapy is selected based on the comparison of step b).

12. The method according to claim 11, wherein the renal therapy is selected from adapting the administration of nephrotoxic medication or treatment, adapting the intake or output of fluid, and adapting the blood pressure,

13. A kit for diagnosing kidney injury in an individual after an acute event or after a surgical intervention or for predicting the risk of an individual to suffer from a kidney injury after an acute event or after a surgical intervention in the future, or for selecting a renal therapy for an individual suffering from or having a risk to suffer from kidney injury after an acute event or after a surgical intervention, comprising: one or more ligand(s) for determining the levels of L-FABP or a variant thereof, preferably further comprising L-FABP or a variant thereof.

14. In vitro use of an antibody binding L-FABP or a variant thereof for predicting or diagnosing a kidney injury in an individual after an acute event or after a surgical intervention or for predicting the risk of an individual to suffer from a kidney injury after an acute event or after a surgical intervention in the future, in a sample isolated from an individual, wherein the level of L-FABP or of a variant thereof is detected in a blood sample isolated within about 10 h after the acute event or after the surgical intervention.

15. A device adapted for carrying out the method of claims 1 to 12, comprising

**EP 2 568 291 A1**

a) an analyzing unit comprising an antibody binding L-FABP, the unit being adapted for determining the level of L-FABP in a first and second sample from an individual; and

b) an evaluation unit for comparing the determined level in the first sample with the level in the second sample whereby a kidney injury in an individual associated with an acute event or a surgical intervention can be diagnosed, the unit comprising a database with ratios of the level of L-FABP in the first sample to the second sample, and a computer-implemented algorithm for carrying out a comparison step.

**Figure 1**

ROC Analysis 2h Post OP

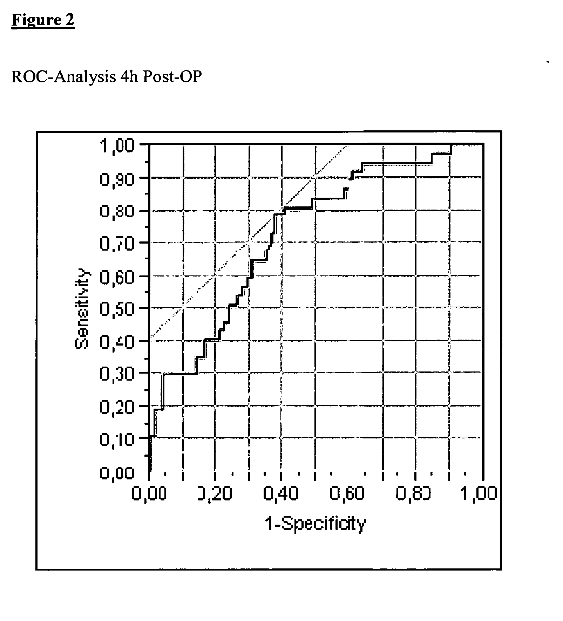

**Figure 2**

ROC-Analysis 4h Post-OP

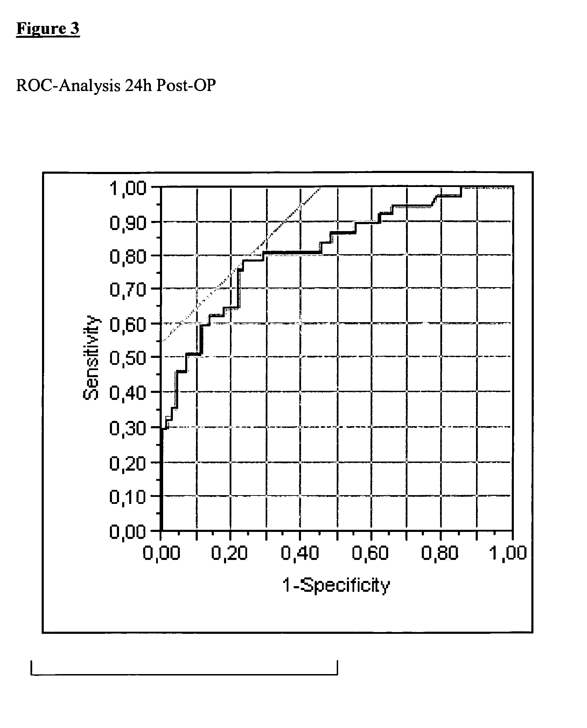

**Figure 3**

ROC-Analysis 24h Post-OP

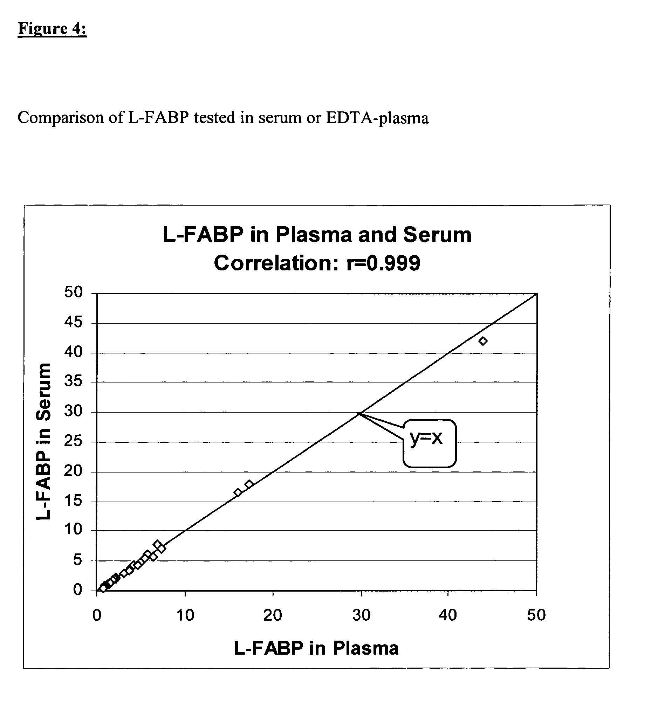

**Figure 4:**

Comparison of L-FABP tested in serum or EDTA-plasma

L-FABP in Plasma and Serum
Correlation: r=0.999

**Figure 5:**

L-FABP in plasma after cardiac surgery in patients suffering from AKI or not suffering from AKI (No AKI)

Europäisches
Patentamt

European
Patent Office

Office européen
des brevets

**EUROPEAN SEARCH REPORT**

Application Number

EP 11 00 7260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | PORTILLA D ET AL: "Liver fatty acid-binding protein as a biomarker of acute kidney injury after cardiac surgery", KIDNEY INTERNATIONAL, NATURE PUBLISHING GROUP, LONDON, GB, vol. 73, no. 4, 1 February 2008 (2008-02-01), pages 465-472, XP002565172, ISSN: 0085-2538, DOI: 10.1038/SJ.KI.5002721 [retrieved on 2007-12-19] * p. 469-471, fig. 8 * | 1,3-8, 13,14 | INV. G01N33/68 |
| X | ATSUKO KAMIJO ET AL: "Urinary liver-type fatty acid binding protein as a useful biomarker in chronic kidney disease", MOLECULAR AND CELLULAR BIOCHEMISTRY, vol. 284, no. 1-2, 11 March 2006 (2006-03-11), pages 175-182, XP55004349, ISSN: 0300-8177, DOI: 10.1007/s11010-005-9047-9 * p. 176, 181; fig. 1a * | 1,2,9-13 | |
| X | WO 2010/125165 A1 (ROCHE DIAGNOSTICS GMBH [DE]; HOFFMANN LA ROCHE [CH]; HESS GEORG [DE];) 4 November 2010 (2010-11-04) * page 34 - page 38 * | 15 | |

TECHNICAL FIELDS
SEARCHED (IPC)

G01N

-/--

The present search report has been drawn up for all claims

| Place of search | Date of completion of the search | Examiner |
|---|---|---|
| Munich | 18 January 2012 | Schalich, Juliane |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

EP 2 568 291 A1

Europäisches Patentamt

European Patent Office

Office européen des brevets

## EUROPEAN SEARCH REPORT

Application Number

EP 11 00 7260

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | STEFANO SALIS ET AL: "Cardiopulmonary Bypass Duration Is an Independent Predictor of Morbidity and Mortality After Cardiac Surgery", JOURNAL OF CARDIOTHORACIC AND VASCULAR ANESTHESIA, vol. 22, no. 6, 1 December 2008 (2008-12-01), pages 814-822, XP55016761, ISSN: 1053-0770, DOI: 10.1053/j.jvca.2008.08.004 * the whole document * * table 4 * | 1-15 | |

TECHNICAL FIELDS SEARCHED (IPC)

The present search report has been drawn up for all claims

| Place of search | Date of completion of the search | Examiner |
|---|---|---|
| Munich | 18 January 2012 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 00 7260

This annex lists the patent family members relating to the patent documents cited in  the above-mentioned European search report.
The members are as contained in the European Patent Office EDP file on
The European Patent Office is in no way liable for these particulars which are merely given for the purpose of information.

18-01-2012

| Patent document cited in search report | Publication date | Patent family member(s) | Publication date |
|---|---|---|---|
| WO 2010125165    A1 | 04-11-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008084003 A
- US 20070042424 A1
- US 5744305 A

### Non-patent literature cited in the description

- **MEHTA et al.** *Circulation,* 2006, vol. 114, 2208-2216
- **BROWN et al.** Annals of Thoracic Surgery. 2008, vol. 86, 4-11
- **KOURLIOUROS et al.** *European Journal of Cardiothoracic Surgery,* 2009
- **SONG et al.** *American Journal of Nephrology,* 2009, vol. 30, 253-260
- **KAMIJO et al.** Urinary liver-type fatty acid binding protein as a useful biomarker in chronic kidney disease. *Mol Cell Biochem.,* March 2006, vol. 284 (1-2), 175-82
- **PORTILLA et al.** *Kidney International,* 2008, vol. 73, 465-472
- **PORTILLA et al.** *The American Journal of Pathology,* 2009, vol. 174, 1154-1159
- **DEVARAJAN et al.** *Informa Healthcare,* 2008, vol. 2, 387-398
- **MEHTA et al.** *Critical Care (London, England,* 2007, vol. 11 (2), 31
- **DOWDY ; WEARDEN.** Statistics for Research. John Wiley & Sons, 1983
- **ZWEIG.** *Clin. Chem.,* 1993, vol. 39, 561-577
- **CHAN et al.** Human liver fatty acid binding protein cDNA and amino acid sequence, Functional and evolutionary implications. *J. Biol. Chem.,* 1985, vol. 260 (5), 2629-2632
- **KJELDSEN et al.** *Journal of Biological Chemistry,* 1993, vol. 268, 10425-10432
- **WAGENER et al.** *Anesthesiology,* 2006, vol. 105, 485-491
- **ICHIMURA et al.** *J Biol Cem,* 1998, vol. 273 (7), 4135-4142
- *Expert Opin. Med. Diagn.,* 2008, vol. 2 (4), 387-398
- **SMITH ; WATERMAN.** *Add. APL. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad Sci. (USA,* 1988, vol. 85, 2444
- **PEARSON ; LIPMAN.** *Proc. Natl. Acad. Sci. (USA,* 1988, vol. 85, 2444
- **NOLAN.** *Trends Biotechnol.,* 2002, vol. 20 (1), 9-12
- **KAMIJO, A et al.** *J Lab Clin Med,* 2004, vol. 143, 23-30
- **KAMIJO, A. et al.** *AJP,* October 2004, vol. 165 (4), 1243-1255